# EUROPEAN PATENT APPLICATION

(11) **EP 0 902 283 A1**
(43) Date of publication of application: **17.03.1999**
(21) Application number: 98115182.2
(22) Date of filing: 12.08.1998
(51) Int. Cl.: G01N 33/00

(54) **Ultra high purity gas analysis using atmospheric pressure ionization mass spectrometry**

(30) Priority: 14.08.1997 US 911068
(71) Applicant: PRAXAIR TECHNOLOGY, INC., Danbury, CT 06810-5113 (US)
(72) Inventor: Goddard, John Burnham, Grand Island, New York 14072 (US); Litwin, Michael Mark, Cheektowaga, New York 14225 (US); Malik, Adam Henry, Lancaster, New York 14086 (US); Sonricker, Michael Harold, North Tonawanda, New York 14120 (US)
(74) Representative: Schwan, Gerhard, Dipl.-Ing.

(57) **Abstract**

A method for measuring the gaseous concentration of at least one selected gas from a plurality of gases comprising hydrogen. The method comprises converting at least one of the selected gas into a measurable gas using a catalyst-oxidizer, and analyzing the measurable gas by atmospheric pressure ionization mass spectrometry to a sensitivity of less than 1 ppb.

## Description

### FIELD OF THE INVENTION

This invention relates to a method and a system for analyzing gaseous components, and more particularly for analyzing the trace components in an ultra high purity gas using atmospheric pressure ionization mass spectrometry.

### BACKGROUND OF THE INVENTION

The semiconductor industry requires ultra high purity (UHP) levels for their use of certain gases. This requirement arises from the need to decrease the dimensions of the line spacing for semiconductor devices. As the line spacings on semiconductor devices become increasingly smaller, the amount of impurities must be maintained in the low parts per billion (ppb) and even parts per trillion (ppt) range to assure device effectiveness. The need for supplying ultra high purity gas with purity levels less than 1 ppb has driven the industry to develop new analytical techniques for measuring gas impurities.

Atmospheric pressure ionization mass spectrometry (APIMS) is an established technique for measuring trace impurities in gases to ppb and sub-ppb levels. For example, in nitrogen or argon base gas, trace water, methane, carbon dioxide, and oxygen can be determined with sub-ppb sensitivity because these gases are ionized very efficiently at a mass where little or no analytical interference exists. Hydrogen presents a special problem for APIMS because it is not efficiently ionized to its parent ion (H₂⁺, mass 2). In nitrogen and gases containing nitrogen, carbon monoxide also presents a special problem because its parent ion (CO⁺, mass 28) has the same mass as nitrogen. This makes the straight forward analysis of all six of these key impurities, namely, hydrogen, methane, water, carbon monoxide, oxygen, and carbon dioxide, to ppb and sub-ppb levels very difficult by APIMS.

Various analytical methods other than APIMS have been used to analyze for hydrogen and/or carbon monoxide gases using catalytic techniques. See, U.S. Patent No. 3,871,827 and European Patent No. 407,818. Although the high sensitivity of the APIMS techniques for determining trace impurities in gases has been recognized, it is believed that there has not been significant work on ultra high purity gas analysis, particularly for a single step detection of a plurality of gases. See, U.S. Patent No. 3,639,757 and European Patent No. 453,190. Other literature relating to APIMS include Ronge et al., "APIMS Technology; Sub-ppb measurement of contaminants in Nitrogen with APIMS: Trace+", Proceedings - Microcontamination 91 Conference and Exposition, San Jose, CA, Oct. 16-18, 1991, pp. 153-167; Kimura et al., "Sub PPB Analysis of Nitrogen Gas by APIMS", Proceedings - Institute of Environmental Sciences 39th Annual Technical Meeting, Vol. 1, Las Vegas, NV, May 2-7, 1993, pp. 14-19; and Ridgeway et al., "Use of Atmospheric Pressure Ionization Mass Spectrometry to Monitor Hydrocarbon Type Impurities in Bulk Nitrogen", Journal of Electrochemical Society 141(9), 2478-2482 (September 1994).

In ultra high purity gas analyses, it is very important to design the analytical system such that the impact due to adsorption and desorption of impurities is minimal. Adsorption and desorption of water is frequently the main concern. The design should be such that changes in the impurity concentrations should be seen quickly and give an equilibrated (steady state) analytical value in a relatively short period of time. Aspects of such a design may include low surface area components, minimum total surface area, constantly flowing gas, and minimum flow changes.

### OBJECTS OF THE INVENTION

It is therefore an object of the invention to provide a method for determining the trace impurities in an ultra high purity gas.

It is another object of this invention to provide a method for determining at least six key impurities in an ultra high purity gas to a sensitivity of less than 1 part per billion with a single instrument.

It is also an object of this invention to provide a system which allows the determination of at least six key impurities in an ultra high purity gas to a sensitivity of less than 1 part per billion with a single instrument.

### SUMMARY OF THE INVENTION

This invention is directed to a method and an analytical system for measuring the gaseous concentration of at least one selected gas from a plurality of gases comprising hydrogen. This method comprises converting at least one of the selected gas into a measurable gas using a catalyst-oxidizer, and analyzing the measurable gas by atmospheric pressure ionization mass spectrometry to a sensitivity of less than 1 ppb.

The catalyst-oxidizer provides oxygen for oxidation and effects a quantitative conversion of the selected gas into the measurable gas. The selected gas may include hydrogen and carbon monoxide, such that the hydrogen is converted to water, and carbon monoxide is converted to carbon dioxide by oxidation in the presence of the catalyst-oxidizer. The plurality of gases comprises hydrogen, carbon monoxide, methane, oxygen, water and carbon dioxide.

This invention is also directed to a catalyst-oxidizer system for converting a selected gas into a measurable gas for measuring the concentration of a gas using atmospheric pressure ionization mass spectrometry. The system comprises a catalyst cell, a heater block, at least one thermocouple and a rod heater.

In various embodiments, the system contains two thermocouples. At least one selected gas is continuously flowing therein. The selected gas is in a base gas which does not interfere with analyzing the measurable gas.

### BRIEF DESCRIPTION OF THE DRAWINGS

Other objects, features and advantages will occur to those skilled in the art from the following description of preferred embodiments and the accompanying drawings, in which:
Fig. 1 is a schematic representation of a catalyst-oxidizer analytical system for ultra high purity gas analysis according to this invention;
Fig. 2 is a side elevation view of catalyst oxidizer cell used in the system of this invention;
Fig. 3 is a top plan view of the heater block as used in the system of this invention;
Fig. 4 is a side elevation view of the heater block as used in the system of this invention;
Fig. 5 is a graphical presentation of the carbon monoxide response at mass 44 (carbon dioxide) as measured using this invention;
Fig. 6 is a linear regression of the graphical presentation of the carbon monoxide response as in Fig. 5.
Fig. 7 is a graphical presentation of the hydrogen response at mass 18 (water) as measured using this invention; and
Fig. 8 is a linear regression of the graphical presentation of the hydrogen response as in Fig. 7.

### DETAILED DESCRIPTION OF THE INVENTION

This invention may be accomplished by a method and an analytical system for measuring the gaseous concentration of at least one selected gas from a plurality of gases comprising hydrogen. This method comprises converting at least one of the selected gases into a measurable gas using a catalyst-oxidizer, and analyzing the measurable gas by atmospheric pressure ionization mass spectrometry to a sensitivity of less than 1 ppb.

The present invention enables the analysis of at least the six main impurities in ultra high purity nitrogen or argon. A single-quadruple APIMS instrument may be used to measure the concentration of at least the six important gases to a sensitivity of less than or about 1 ppb.

In this invention, the direct analysis of hydrogen as water and carbon monoxide as carbon dioxide is possible. Water and carbon dioxide have a mass of 18 and 44, respectively. At these masses, water and carbon dioxide have a high sensitivity by APIMS analysis. Also, these masses are essentially interference-free, such that the background count levels (cps) at masses 18 and 44 are attributed to the water and carbon dioxide, rather than to the presence of other ions. This avoids the conventional problem such as that represented by measuring hydrogen at mass 29 (N₂H⁺) in which ¹⁵N¹⁴N⁺ interferes. Other proton donors such as water may contribute to the mass 29 N₂H⁺ ion, further confusing the analysis of molecular hydrogen by that route. Similarly, a conventional problem exists for measuring carbon monoxide at mass 12 (C⁺), because other carbon bearing compounds will decluster to C⁺ and all will have different sensitivities, making the C⁺ analytical data difficult to interpret unambiguously.

Additionally, by using catalytic oxidation, the APIMS sensitivities (cps/ppb) for hydrogen measured as water at mass 18 and for carbon monoxide measured as carbon dioxide at mass 44 are significantly greater than the sensitivities at masses 29 and 12, respectively. The higher sensitivity and lower interference lead to lower calculated detection limits.

A catalyst-oxidizer, i.e., a material which both provides oxygen for oxidation and effects a quantitative conversion of hydrogen to water and carbon monoxide to carbon dioxide without having to add oxygen to the sample gas stream, may be used in this invention. Copper oxide wire coated with about 0.5 weight percent palladium was used to provide both the oxygen needed and the catalytic action to effect the oxidation of both hydrogen and carbon monoxide in the range 200°C-400°C.

No other products are produced because the catalyst is operated at a temperature in which methane would not oxidize. No oxygen is required in the gas stream, though periodically, the catalyst-oxidizer may be regenerated with a gas stream containing some oxygen (the amount not being critical, but optimally 1% oxygen or more, with the balance typically being nitrogen. Pure oxygen may also be used).

The catalyst is prepared by soaking copper oxide wire with a methanol solution of palladium nitrate, evaporating and roasting at 300°C for one hour to convert the palladium nitrate to palladium oxide/palladium metal. Other methods for preparing this catalyst may be used. The use of copper oxide without palladium as a catalyst-oxidizer for hydrogen and carbon monoxide analyses by the Orsat method has been known in the art. See, V.J. Altieri, "Gas Analysis & Testing of Gaseous Materials", pp. 174,181, American Gas Association, Inc., New York (1945).

Besides palladium, platinum or other platinum group metals may be used for the coating material. Other metal oxides such as cobalt oxide, which can give up oxygen to carbon monoxide and/or hydrogen, may be used. A metal oxide that yields its oxygen at relatively low temperature, e.g., less than about 450°C-500°C, is preferable. Palladium (oxide) or platinum may be employed to enhance the conversion rate. The palladium (oxide) or platinum may be used directly, or may be deposited on a support such as silica, alumina or titania. The catalyst should not have a high capacity for water or carbon dioxide such that there is a significant delay in the response of the analytical method when a concentration change occurs or such that there are high and variable levels of water and carbon dioxide outgassing from the catalyst into the incoming sample gas.

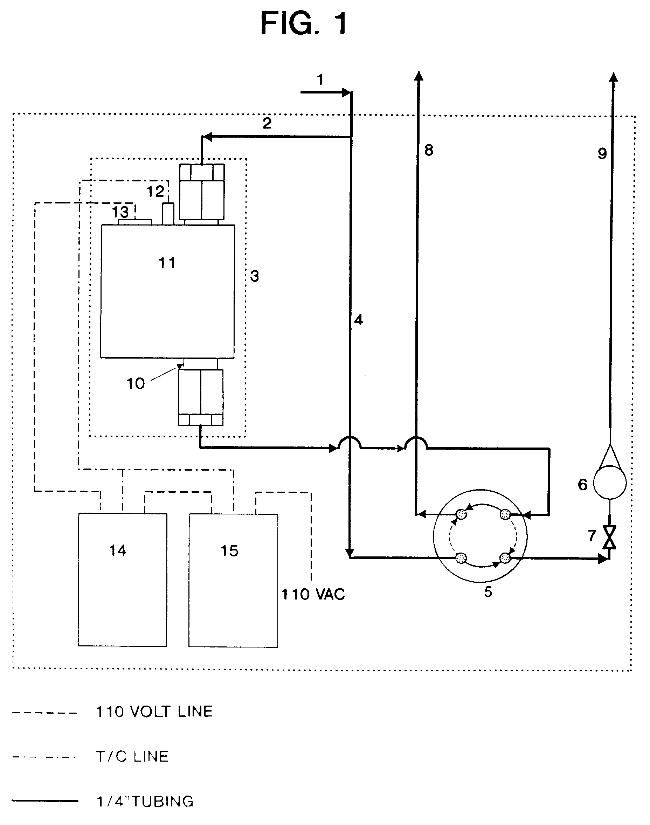

Turning now to the figures, Fig. 1 provides a schematic of the catalyst-oxidizer analytical system. Referring to Fig. 1, the sample or calibration gas enters through stainless steel inlet line 1, for which the flow is controlled by a mass flow controller system. The flow is then divided between "through-catalyst" line 2 containing insulated catalyst oxidizer system 3, and the "bypass-catalyst" line 4. Each of these gas flows enters a port on four-port, two position manual switching valve 5 which in the mode depicted directs the "through-catalyst" flow line 2 to the APIMS analyzer through line 8 and bypass-catalyst flow line 4 to vent line 9 after passing through a flow indicator (rotameter) 6 which is equipped with a flow control valve 7. The proportion of inlet flow 1 reaching the catalyst or bypassing the catalyst is controlled by valve 7. The mineral-wool insulated catalyst-oxidizer system 3 contains catalyst cell 10, heater block 11, two thermocouples 12, and rod heater 13. Temperature controller 14 regulates the temperature of the catalyst-oxidizer, and over-temperature controller 15 serves as a safety shut-off if the block temperature exceeds a preset value.

A gas constantly flows through the catalyst-oxidizer system 3, bypass line 4 and all ports of the switching valve 5. The switching valve 5 may be switched to the flow position as indicted by the dotted lines, i.e., the "through-catalyst" flow is directed to the vent and the "bypass-catalyst" flow is directed to the APIMS analyzer. This is useful for determining the amount of water and carbon dioxide in the sample gas before any oxidation of carbon monoxide or hydrogen. In the catalyst bypass mode, a portion of the sample gas flows over the catalyst and out to vent. It is important to maintain a gas flow over the catalyst at the temperature of the catalyst (200°C-400°C) so that any catalyst outgas products, especially moisture, are continuously removed. Otherwise, slugs of moisture to the APIMS may result once valve 5 is switched to the through-catalyst analysis position, which significantly increases the time needed to reach a steady state moisture value representative of the gas sample. Ideally, to minimize the impact of changes in outgassing equilibrium, the through-catalyst-to-vent flow rate should match the bypass-catalyst-to-APIMS flow rate. Flow rate adjustments can be made with valve 7. Thus, when the valve 5 is switched to the through-catalyst-to-APIMS mode, and the flow to the APIMS remains the same, any outgassing from the catalyst up to valve 5 will not change. This leads to a more rapid attainment of equilibrium carbon dioxide and (especially) moisture levels, which in turn speeds up the analysis.

The operating temperature is limited to within 200°C-400°C for this invention. The requirement is that the operating temperature of the catalyst on the low end be sufficient to oxidize hydrogen to water and carbon monoxide to carbon dioxide essentially quantitatively. The higher temperatures should be used only to improve response time; the upper limit should be that at which no significant methane oxidation can occur.

The catalyst oxidizer is placed in a device which can be heated to 200°C-400°C and the temperature maintained essentially constant. This is accomplished readily with a stainless steel tube to hold the catalyst, with screens or preferably filter disks of typically 10-100 µm pore size to prevent catalyst particulates from being transported out of the catalyst container. The tube preferably is heated from the outside by electric heaters that are controlled by a temperature controller. An independent over temperature protection device also is preferred to be present to shut off the heaters if a certain temperature is exceeded.

The ideal amount of catalyst to use is the minimum necessary to effectuate the total conversion of carbon monoxide to carbon dioxide, and hydrogen to water at the flow rate and temperature chosen. The more catalyst used, the longer the response time for the hydrogen analysis, since the moisture product is retained by the catalyst for a time until the catalyst becomes saturated with water at the conditions chosen. The water then is evolved and reaches equilibrium with the incoming hydrogen so that there is a one-to-one molar correspondence. The carbon dioxide created reaches equilibrium very quickly compared to water, so there is little delay in reaching a one-to-one correlation with incoming carbon monoxide. For the palladium-coated copper oxide catalyst, 1.27 cm³ (3.85g) was sufficient for quantitative conversion of carbon monoxide to carbon dioxide, and hydrogen to water at 1900 mL/min nitrogen flow at a catalyst temperature of 325°C-350°C. Using ten times that amount of catalyst increased the response time for hydrogen analysis significantly. A preferred method includes a means to direct the sample to the APIMS while bypassing the catalyst so that the carbon dioxide and water already present in the sample stream can be determined. These values are then subtracted from those obtained when the sample passes through the catalyst in order to calculate the hydrogen and carbon monoxide in the original sample.

There is no particular limitation in the flow rates (space velocities) through the catalyst in this invention. Preferably, the space velocity is the highest one that will give essentially quantitative conversion at the conditions chosen. The faster flows will result in quicker equilibration and hence more rapid response during the analysis.

Other base gases beside nitrogen can be utilized in the invention, especially those where nitrogen is a likely contaminant that would interfere with the analysis of CO at mass 28. Hydrogen also is generally difficult to analyze as H₂⁺ (mass 2) since its proclivity to protonate other neutrals, such as N₂ or Ar, can greatly affect the quantitative analysis. For instance, in argon base gas, hydrogen can form ArH⁺. However, water and other proton donor molecules may protonate as well. Hence, the invention is advantageous for argon and the other noble gases, particularly helium and neon. Since the nitrogen ionization potential is lower than the ionization potential of helium and neon, nitrogen will ionize extensively, interfering with carbon monoxide analysis at mass 28. For the analysis of trace hydrogen, the invention would be useful in any gas where water has a lower ionization potential than the base gas. For practical purposes, these gases include helium, neon, argon and krypton.

Preferably, a calibration gas is run through the catalyst under the same conditions as that for the sample prior to actual measurement of the sample gas. From the calibration curve, which should be linear, a slope (sensitivity in cps/ppb) and intercept (background) are obtained at both mass 18 (H₂O) and mass 44 (CO₂). The "zero gas" may be a sample gas run through, for example, 5A molecular sieve to remove carbon dioxide and water. The "zero gas" also may be another independently purified gas stream rather than the sample stream. Normally, the APIMS is calibrated with the "zero gas" into which small concentrations of impurities are blended, e.g., by a mass flow control blending system. See, commonly assigned U.S. Patent No. 5,214,952 to Leggett et al., which is incorporated herein by reference. As a result, the "zero gas" background reading is without the impurities.

### EXAMPLE 1

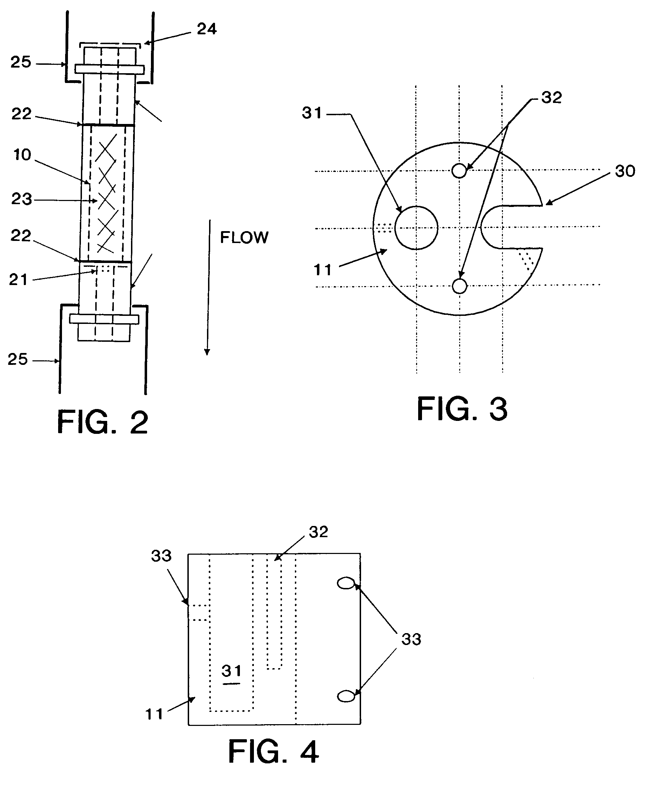

Turning now to Fig. 2, a stainless steel catalyst vessel 10 was made from 3/8" stainless steel tubing to which was welded by welds 22 on the gas inlet side one-half of a face seal close coupling 20 and on the exit end a 10-micron filter 21 with the other half of the face seal close coupling 20. The internal volume 23 was filled with 1.27 cm³ (3.85 g) of copper oxide wire previously coated with 0.5% palladium. (The palladium coating was obtained by adding 3.03 g Pd(NO₃)₂ dissolved in 100 mL methanol to 280 g copper oxide in an evaporating dish, slowly evaporating with stirring, then roasting the material for one hour at 300°C.) The catalyst was sealed in with a 60-µm disk filter gasket 24. The catalyst oxidizer vessel 10 is connected to gas line 2 with 1/4-inch face seal female nuts 25.

Turning to Figs. 3 and 4, the catalyst oxidizer cell was placed in slot 30 of a stainless steel block 11, which also contained a cartridge heater port 31 and two thermocouple ports 32. The thermocouple ports were spaced equidistant from the cell and the heater rod surface. The cartridge heater and catalyst container were held by set screws 33. The whole system was insulated with mineral wool and placed in a box for control.

### EXAMPLE 2

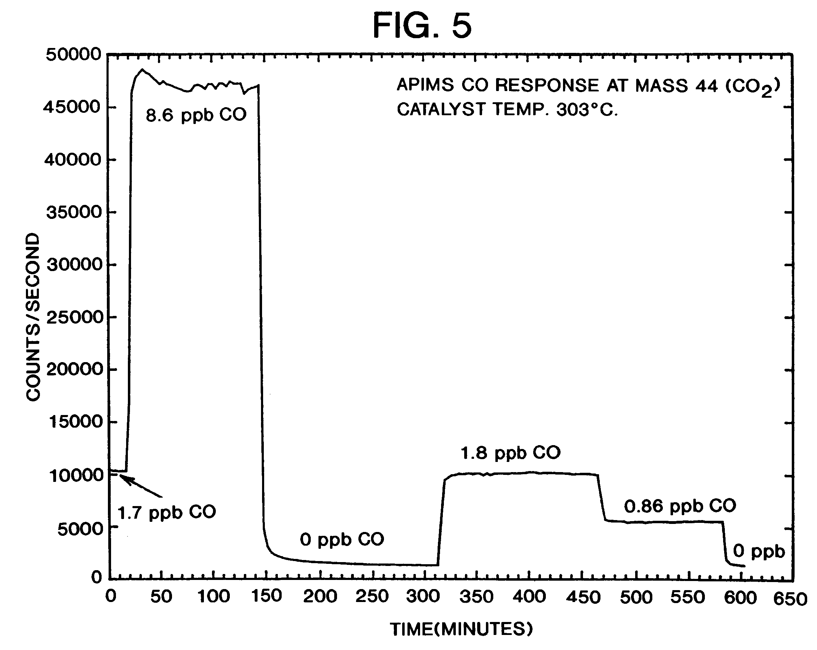

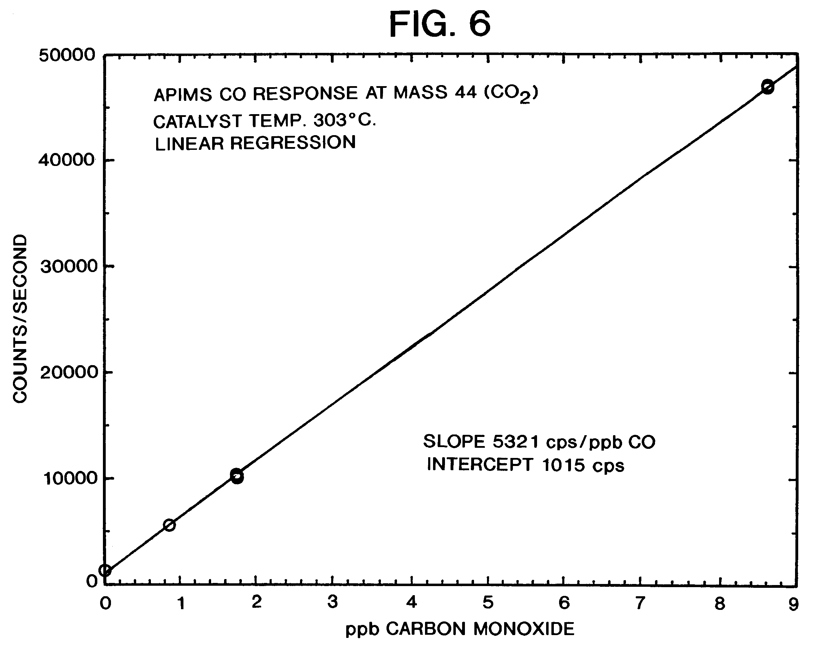

A nitrogen calibration gas containing 300 ppb carbon monoxide was blended with a nitrogen zero gas containing less than 1 ppb of carbon monoxide. Any carbon dioxide present in the calibration gas was removed by sodium hydroxide on nonfibrous silicate carrier (Ascarite II, trademark of Thomas Scientific, Swedesboro, NJ) and 5A molecular sieve columns and that present in zero gas was removed by 4A molecular sieve and heated metal getter purifiers to levels of less than 1 ppb of carbon dioxide. Blending was done in a system similar to that described in U.S. Patent No. 5,214,952. Blends were put through the catalyst system described in Example 1 at about 1500 sccm at a catalyst temperature of 303°C. Fig. 5 shows the response at mass 44 (carbon dioxide) to the amount of carbon dioxide added, and Fig. 6 is a regression plot of the data taken at the end of the (equilibrated) level before the next change in concentration. The conversion was quantitative, the response was linear and good sensitivity was achieved (5321 counts/second/ppb carbon monoxide). The detection limit was defined by the International Union of Pure and Applied Chemistry (IUPAC) as three times the noise, i.e., three times the standard deviation of the count rate for zero gas, was 0.005 ppb carbon monoxide. The zero gas background was also low (averaging 1291 counts/second, equivalent to about 0.24 ppb carbon monoxide).

### EXAMPLE 3

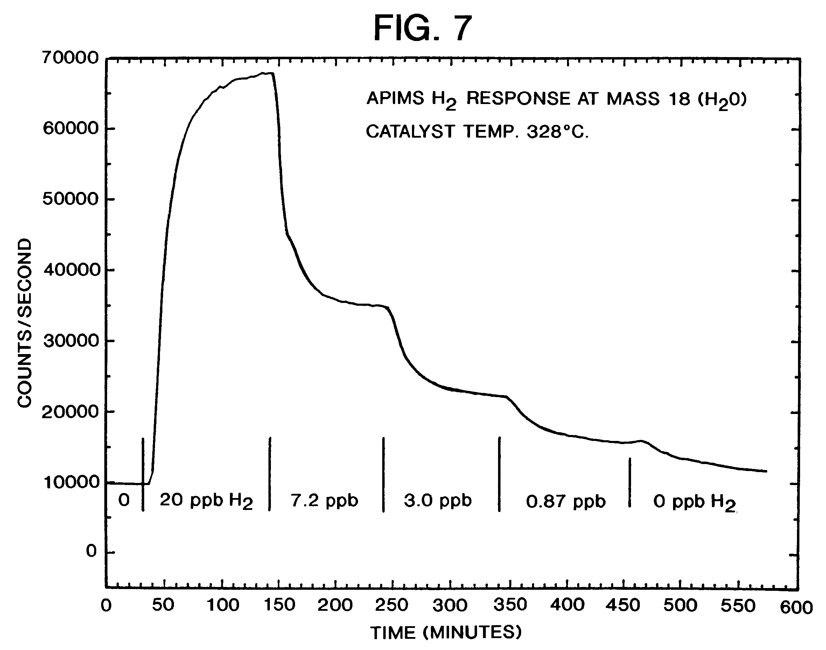

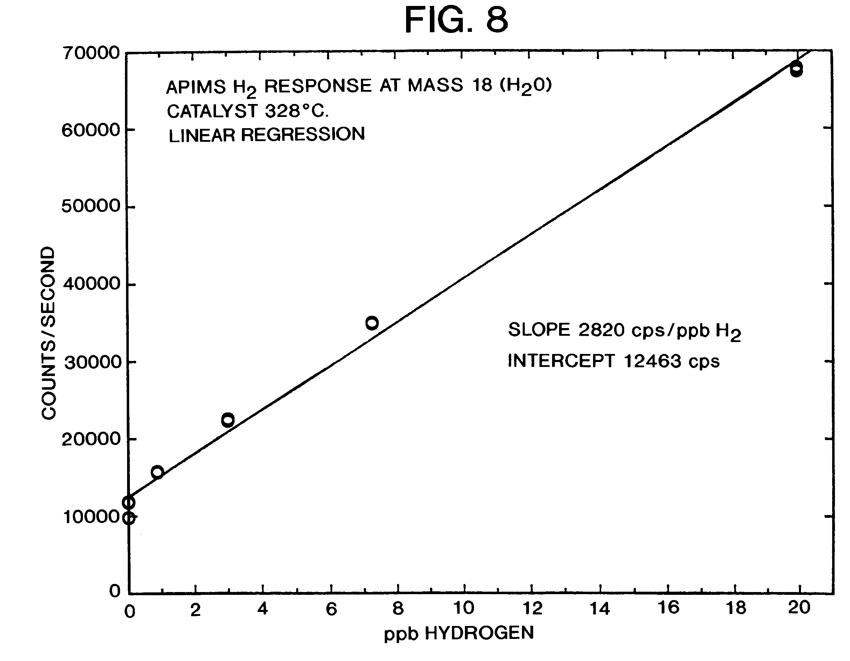

A nitrogen calibration gas containing 800 ppb hydrogen was blended with a nitrogen zero gas containing less than 4 ppb hydrogen. Any water in the calibration gas was removed by a 5A molecular sieve column and water and hydrogen were removed from the zero gas by 4A molecular sieve and a heated metal getter purifier. Blending was done as in Example 2. The flow through the catalyst was about 1900 sccm at a catalyst temperature of 328°C. Runs under similar conditions gave quantitative conversion to water. Fig. 7 shows the response at mass 18 (water) to the amount of hydrogen added, and Fig. 8 is a regression plot of the data taken at the end of the (equilibrated) level before the next change in concentration. Good sensitivity was achieved (2820 cps/ppb hydrogen). The IUPAC detection limit was 1.1 ppb hydrogen. The zero gas background count averaged 10795 counts/second, equivalent to 3.8 ppb hydrogen.

### EXAMPLE 4

A blend of nitrogen containing 25 ppb methane and 3.6 ppb carbon dioxide was fed to the above catalyst at 1000 ccm at 303°C, 353°C and 403°C. The amount of methane lost and amount of carbon dioxide gained on passing through the catalyst vs. bypassing the catalyst was measured by APIMS. Table 1 shows the results.

**TABLE 1**

| Temp. °C | ppb CH₄ lost | ppb CO₂ gained |
|---|---|---|
| 303 | 0 | 0 |
| 353 | 1.8 ± 0.2 | 0.9 ± 0.2 |
| 403 | 1.0 ± 0.5 | 1.7 ± 0.8 |

At 303°C, the amount of methane lost and carbon dioxide gained was statistically zero. At 353°C and 403°C, slight methane losses and carbon dioxide gains were noted, but it represented 7% or less of the methane present. Thus, 303°C and below is best, but even as high as 403°C, the amount of methane oxidized is very small and may be acceptable analytically depending upon the amount of methane present in the sample in comparison to the amount of carbon monoxide.

### EXAMPLE 5

Blends of nitrogen containing 1-21 ppb hydrogen and 0.4-8 ppb carbon monoxide were passed through the above catalyst at 1800 ccm at 202°C, 253°C, 303°C, 352°C and 402°C. Plots of APIMS response in counts/second were linear over this range for water at mass 18 and carbon monoxide at mass 44, and the conversion was essentially complete and unaffected by temperature in the range stated.

Specific features of the invention are shown in one or more of the drawings for convenience only, as each feature may be combined with other features in accordance with the invention. Alternative embodiments will be recognized by those skilled in the art and are intended to be included within the scope of the claims.

## Claims

1. A method for measuring the gaseous concentration of at least one selected gas from a plurality of gases comprising hydrogen, said method comprising converting at least one of said selected gas into a measurable gas using a catalyst-oxidizer, and analyzing said measurable gas by atmospheric pressure ionization mass spectrometry to a sensitivity of less than 1 ppb.

2. The method of claim 1 wherein said catalyst-oxidizer provides oxygen for oxidation and effects a quantitative conversion of said selected gas into said measurable gas.

3. The method of claim 1 wherein said plurality of gases is in a base gas which does not interfere with analyzing said measurable gas.

4. The method of claim 1 wherein hydrogen is converted to water in the presence of said catalyst-oxidizer.

5. An analytical system for measuring the gaseous concentration of at least one selected gas from a plurality of gases comprising hydrogen, said system comprising a catalyst-oxidizer adapted to convert at least one of said selected gas into a measurable gas for measuring the gaseous concentration by atmospheric pressure ionization mass spectrometry to a sensitivity of less than 1 ppb.

6. The system of claim 5 wherein said catalyst-oxidizer provides oxygen for oxidation and effects a quantitative conversion of said selected gas into said measurable gas.

7. The system of claim 5 wherein said plurality of gases is in a base gas which does not interfere with analyzing said measurable gas.

8. The system of claim 5 wherein hydrogen is converted to water in the presence of said catalyst-oxidizer.

9. A catalyst-oxidizer system for converting a selected gas into a measurable gas for measuring the concentration of a gas using atmospheric pressure ionization mass spectrometry, said system comprising a catalyst cell, a heater block, at least one thermocouple and a rod heater.

10. The catalyst-oxidizer system of claim 10 wherein said system contains two thermocouples.
